# EUROPEAN PATENT SPECIFICATION

(11) **EP 1 990 012 B1**
(45) Date of publication and mention of the grant of the patent: **30.06.2010**
(21) Application number: 07447031.1
(22) Date of filing: 09.05.2007
(51) Int. Cl.: A61B 10/02

(54) **A medical tool arrangement**
Anordnung medizinischer Werkzeuge
Agencement d'outil médical

(43) Date of publication of application: 12.11.2008
(73) Proprietor: Janssens, Jaak Ph., 3500 Hasselt (BE)
(72) Inventor: Janssens, Jaak Ph., 3500 Hasselt (BE)
(74) Representative: Bird, Ariane

(56) References cited:
- EP-A- 1 250 890
- WO-A-02/22023
- WO-A-97/20504
- WO-A-02/065919
- WO-A-2007/051509
- US-A- 5 526 822
- US-A- 6 083 237
- US-A1- 2004 002 664
- US-A1- 2006 122 535

## Description

### Technical field of the invention

The present invention relates to medical tool arrangements comprising a tool assembly having a plurality of tools. The medical tool arrangements may be medical tool arrangements for sampling tissue from human or animal body, using biopsy needles or needle assemblies.

### Background of the invention

Biopsy needle assemblies are already well known in different forms. As an example, US2003/0114773A1 describes a biopsy needle assembly comprising an outer cannula for insertion in a tissue of which the front end has a cutting edge for cutting the tissue sample. The biopsy needle assembly comprises further a hollow biopsy needle located inside and coaxially with the cannula, at the front end being provided with a tissue receiving means, such as a helical screw. The tissue receiving means cooperates with the cutting edge of cannula for receiving cut tissue sample. The biopsy needle assembly further can comprise an inner needle or localisation needle, having a front end and a back end, this inner needle being located inside and coaxially with the hollow biopsy needle. In order to be able to sample tissue, the cannula, hollow biopsy needle and inner needle are mutually axially and rotatably moveable with respect to each other.
It is known to create a vacuum at the end of the biopsy needle when brought into the tissue to be sampled, as e.g. described in US5526822. The biopsy needle assembly is to be connected to an external vacuum- source by means of appropriate tubing. Another device for sampling tissue from human or animal body, using a biopsy needle assembly is shown in US2005/0165328A1. This biopsy needle, once brought in the tissue to be sampled, uses a vacuum pressure-creating device connected to a proximal end of the biopsy needle brought into the tissue. This vacuum attracts the surrounding tissues into the receiving room prior to cutting.

The presently known vacuum assisted biopsy needle assemblies require a significant air volume to be extracted to place the needle cavities under vacuum or underpressure, which air volume is present in the tubing, between the needle and an external vacuum unit, or within a hand held unit. This requires significant size increase of the vacuum unit. It is also a disadvantage of the presently known vacuum assisted biopsy needle assemblies that an additional manipulation or action of the physician is needed in order to activate the vacuum at the end of the biopsy needle. As medical interventions become more and more complex, such additional action to activate the vacuum may easily be overlooked. It is as well a disadvantage that most often, when a physician is to take several consecutive tissue samples, the biopsy needle assembly is to be brought in as many times as tissue samples are needed. This obviously causes discomfort for both patient and physician, may result in more damage caused to the patient by the consecutive penetration of the body and may cause seeding of diseased, e.g. tumour cells along the extraction pathway.
It is also a disadvantage of presently known assemblies, that driving means such as motors are located in the hand hold device, which may cause interference, such as EMI interference, with the other apparatuses used during the medical manipulation, e.g. scanning equipment such as MRI scanners which are used in conjunction with the biopsy needle to ensure accurate placing of the needle tip.

### Summary of the invention

It is an object of the present invention to provide an improved medical tool arrangement comprising a tool assembly having a plurality of tools.
It is an advantage of the present invention that consecutive tissue samples may be taken without the need of removing the biopsy needle assembly after each sample been taken. It is as well an advantage of the present invention that EMI interferences, e.g. from the driving motors may be avoided.
It is also an advantage of the present invention that the medical tool arrangement, optionally a medical tool arrangement for taking tissue sample, is provided in a hand-held casing, which dimensions and weight of the hand-held casing may be reduced. The reduced dimensions and weight make the hand-held casing useful for handsome handling, especially in combination with other apparatuses used during the medical manipulation, e.g. scanning equipment such as MRI scanners which are used in conjunction with the medical tool arrangement, such as a medical tool arrangement comprising biopsy needles for taking tissue sample, to ensure accurate placing of the medical tools.

The invention is defined in the independent claim 1. Preferred embodiments are defined in dependent claims 2-11. An arrangement according to the preamble of claim 1 is known from WO-A-02/22023.

According to the present invention, a medical tool arrangement for taking tissue sample is provided. The medical tool arrangement comprises a hand-held casing. The hand-held casing comprises a biopsy needle assembly having a plurality of biopsy needles. The hand-held casing further comprises a driving mechanism for moving the plurality of biopsy needles independently of each other. The medical tool arrangement is for use with a mechanical energy source remote from the hand-held casing. The mechanical energy source is couplable to the driving mechanism of the hand-held casing by means of at least a first and a second mechanical energy transferring means. At least one of the first and second mechanical energy transferring means has a length and an axial direction and comprises an energy transferring medium suitable for transferring mechanical energy by making a translational movement longitudinally along its axial direction.

The biopsy needles comprise:
- an outer cannula having a front end and a back end. The front end is for insertion in a tissue. The front end has a cutting edge for cutting a tissue sample;
- a hollow biopsy needle located inside and coaxially with the cannula. The hollow biopsy needle has an inner cavity and has a front end and a back end. The front end is provided with a tissue receiving means. The tissue receiving means cooperates with the cutting edge of cannula for receiving a cut tissue sample
Optionally the biopsy needle assembly may further comprise an inner needle having a front end and a back end. The inner needle is located inside and coaxially with the hollow biopsy needle;

The driving mechanism may be adapted for:
o at least axially displacing forwards and backwards the cannula relative to the hollow biopsy needle and the inner needle;
o at least axially displacing forwards and backwards the hollow biopsy needle relative to the cannula and the inner needle;
o enabling or disabling the inner needle to move in axial direction along with the hollow biopsy needle when the hollow biopsy needle is axially moved.

The driving mechanism may comprise a driving means for enabling or disabling the inner needle to move in axial direction along with the hollow biopsy needle when the hollow biopsy needle is axially moved. The at least one of the first and second mechanical energy transferring means suitable for transferring mechanical energy by making a translational movement may be for providing mechanical energy to the driving means for enabling or disabling the inner needle to move in axial direction along with the hollow biopsy needle when the hollow biopsy needle is axially moved.
The advantage of the medical tool arrangement according to the present invention is that tissue samples, such as several consecutive tissue samples, may be taken without the need of removing the biopsy needle assembly after each sample has been taken. It is as well an advantage that EMI interferences, e.g. from the driving motors, may be reduced or even avoided.
It is also an advantage of the medical tool arrangement according to the present invention that the biopsy needle assembly is provided in a hand-held casing, which dimensions and weight of the hand-held casing may be reduced. The reduced dimensions and weight make the hand-held casing useful for handsome handing, especially in combination with other apparatuses used during the medical manipulation, e.g. scanning equipment such as MRI scanners which are used in conjunction with the biopsy needle to ensure accurate placing of the needle tip.

The medical tool arrangement for taking tissue sample may have as an advantage that neither recipients nor electrical vacuum aspirators are used to provide a suction or vacuum pressure to the end of the biopsy needle. It is also an advantage of some embodiments of the present invention, that the suction or vacuum pressure is created automatically with the normal manipulations of the biopsy needle, i.e. independently from special actions taken by the physician in order to create the suction or vacuum at the end of the biopsy needle. It is also an advantage of the medical tool arrangement for taking tissue sample according to some of the embodiments of the present invention, comprising a biopsy needle assembly that the volume of trapped air to be removed before suction or vacuum is applied may be reduced. This may result in a vacuum unit of reduced size.

An advantage is the combined action of a helix and suction or a vacuum to localize the specimen into the receiving room during and before cutting.
Another advantage is the lack of noise during the biopsy procedure. Normal vacuum assisted biopsies make a suction noise in the tubing and vacuum generator.

The at least one of the first, and second and optionally a third mechanical energy transferring means suitable for transferring mechanical energy by making a translational movement may be a flexible shaft. Optionally the first, second and third mechanical energy transferring means may be three flexible shafts.
At least one of the flexible shafts may comprise a cable, such as a transmission cable, being the medium suitable for transferring mechanical energy by making a translational, i.e. a backwards and forwards movement. The cable, such as the transmission cable, may be suitable for making a rotational movement as well.

Some or all three transmission cables may be suitable for making a rotational movement.

The mechanical energy source may be one motor coupled to the transmission cables by means of an appropriate gearbox. Alternatively, the mechanical energy source may be a plurality of motors, e.g. three motors, being coupled to the transmission cables by means of one or more gearboxes. The one or more motors may be activated, and the one or more gearboxes may be controlled by a control unit.

The at least one of the first and second and optionally third mechanical energy transferring means suitable for transferring mechanical energy by making a translational movement may be a hydraulic tube comprising a hydraulic fluid. Optionally the first, second and third mechanical energy transferring means may be three hydraulic tubes comprising a hydraulic fluid.
The hydraulic tube comprises a hydraulic fluid, such as water, diethylhexylphthalate, mineral oils or alike, being examples of fluids suitable for transferring mechanical energy by making a translational movement, i.e. flowing within the hydraulic tube.
The hand-held casing of the medical tool arrangement may further comprise means to translate the translational, i.e. the backward and/or forward movement, of the hydraulic fluid into rotation of driving means such as gear wheels, used to rotate the medical tools, e.g. a rotary drill, a rotary cutting device, a cannula and/or a hollow biopsy needle.
The mechanical energy source may comprise one or more hydraulic pumps coupled to the hydraulic tube or tubes by means of an appropriate valve system. A control unit may control the hydraulic pump and the valve system.

The at least one of the first and second and optionally third mechanical energy transferring means suitable for transferring mechanical energy by making a translational movement may be a compressed gas tube suitable for conducting compressed gas. Optionally the first, second and third mechanical energy transferring means may be three compressed gas tubes suitable for conducting compressed gas.
Compressed gas is to be understood as gas under pressure greater than that of the atmosphere.
The compressed gas tube may comprise compressed air or any other suitable and medically allowed gas suitable for transferring mechanical energy by making a translational, usually a forward, movement.
The hand-held casing of the medical tool arrangement may further comprise means to translate the translational movement of the compressed gas into movement of the tools. This may be done by rotation of driving means such as gear wheels, used to rotate the medical tools, e.g. a rotary drill, a rotary cutting device, a cannula and/or a hollow biopsy needle.
The mechanical energy source may comprise one or more gas compressors and/or one or more compressed gas buffers. The mechanical energy source may be provided with compressed gas from a compressed gas distribution network via a compressed gas intake. The compressors, or compressed gas buffers or the compressed gas intake is coupled to the compressed gas tube or tubes by means of an appropriate valve system. A control unit may control the compressors and/or the valve system.

The hand-held casing further may comprise a control panel for controlling the driving mechanism.The mechanical energy source may comprise a control unit for activating the first, second and optionally a third mechanical energy transferring means. The medical tool arrangement further may comprise a signal transmitting connection between the control panel and the control unit for converting the signal of the control panel to activation or deactivation of one or more of the mechanical energy transferring means.
The signal transmitting connection between the control panel and the control unit may be a wireless connection, or may be provided by a signal cable, coupling the control panel to the control unit. The control unit is suitable to activate each of the first, second and third mechanical energy transferring means separately. The control unit may further control optional gear boxes or valve systems, in order to control the timing and amount of energy provided to each of the mechanical energy transferring means according to signals obtained from the control panel and/or according to preset routines.

The medical tool arrangement comprises a biopsy needle assembly comprising a hollow biopsy needle, a cannula and an inner needle. The hollow biopsy needle optionally is rotatably mounted around common axis of cannula, hollow biopsy needle and inner needle. Although any kind of hollow biopsy needle may be used, a hollow biopsy needle comprising a spirally shaped tissue receiving means at its tip is a preferred embodiment. Such tissue receiving means, cooperating with a cannula having a cutting edge at its front end, provides tissue samples with reduced damage.
The hollow biopsy needle may be provided with a cylindrical back end. The cylindrical back end of the hollow biopsy needle may be rotated by a suitable driving mechanism of the medical tool arrangement of the present invention, comprising e.g. friction wheels or gears contacting the cylindrical outer surface and forcing a cylindrical back to rotate. Optionally however, the cylindrical back end of this rotatable hollow biopsy needle is provided with a non-slip drive means, e.g. a contact wheel or gear for engaging without slippage with a counter wheel or gear in order to rotate the hollow biopsy needle. Most preferred, the contact wheel and counter wheel are gear wheels. All moving parts of the biopsy needle assembly may be made of plastic materials to save weight. Alternatively metal parts may be used, e.g. for gear wheels, to provide longer life and to reduce the volume of the parts.

Such gear wheels enable provision of a very accurate radial positioning of the hollow biopsy needle. They may also cause the cooperating gear wheel of the cylindrical back end of the hollow biopsy needle to be blocked.
Optionally the inner needle is rotatably mounted around the common axis of cannula, hollow biopsy needle and inner needle. The cylindrical body of the inner needle may be rotated by the driving mechanism, such as by means of a wheel or gear contacting the cylindrical body and forcing the cylindrical body to rotate. Optionally however, the cylindrical body of this rotatable inner needle is provided with a non-slip drive means, e.g. a contact wheel or gear for engaging without slippage with a counter wheel or gear in order to rotate the inner needle. Most preferred, the contact wheel and counter wheel are gear wheels.
Such gear wheels enable to provide a very accurate radial positioning of the inner needle. They may also cause the cooperating gear wheel of the cylindrical body of the inner needle to be blocked.
Optionally, the cylindrical body is provided with an outer screw thread, and the inner side of the cylindrical back end of the hollow biopsy needle includes an inner screw thread engaging with the screw thread of the cylindrical body. In order to provide a piston-cylinder vacuum unit, the cylindrical body comprises means to provide an airtight coupling of cylindrical body and cylindrical back end, such as a seal or joint. Such coupling of the cylindrical body of the inner needle with the cylindrical back end of the hollow biopsy needle provides a well defined axial displacement of the inner needle and hollow biopsy needle when one of the two needles is rotated, while the other is in a fixed radial position.

The driving mechanism may comprise a first and a second wheel, optionally gear wheels. The first wheel is provided for engaging and rotating the hollow biopsy needle. The second wheel is provided for engaging and allowing the inner needle to rotate along simultaneously with the hollow biopsy needle. The first wheel is provided with mechanical energy by at least one of the mechanical energy transferring means, optionally a mechanical energy transferring means being a flexible shaft providing mechanical energy by means of a rotation of a transmission cable being part of this mechanical energy transferring means.

The driving mechanism is suitable for enabling and disabling, i.e. preventing the inner needle from moving in an axial direction when hollow biopsy needle is moved axially. Therefore the driving mechanism comprises a driving means for enabling and disabling, i.e. preventing, the inner needle from rotating when the first wheel is engaging and rotating the hollow biopsy needle. Especially in the case when the inner needle and the hollow biopsy needle are coupled by means of cooperating and engaging screw threads, it is sufficient to rotate the hollow biopsy needle while the inner needle is blocked, this is prevented to rotate along with the hollow biopsy needle. This driving means is provided with mechanical energy by a second of the mechanical energy transferring means, optionally the at least one mechanical energy transferring means suitable for transferring mechanical energy by making a translational movement. By making the translational movement, a means for preventing the inner needle to rotate may be engaged. Optionally this mechanical energy transferring means is a flexible shaft providing mechanical energy by means of a translation of a transmission cable being part of this mechanical energy transferring means.

The driving mechanism is suitable for axially moving or displacing the cannula forwards and backwards. The driving mechanism may comprise a a third wheel, optionally a gear wheel, for engaging and rotating the cannula. Especially in the case the cannula and the hollow biopsy needle are coupled by means of cooperating and engaging screw threads, as will be set out further, it is sufficient to rotate the cannula while the hollow biopsy needle is blocked in order to move the cannula relative to the hollow biopsy needle and the inner needle.
This may occur when the hollow biopsy needle engages the first wheel, which is however blocked because of the first of the mechanical energy transferring means is provided with energy to keep the first wheel stayed in a fixed position, while the cannula is to rotate.
Due to the screw threads cooperating, the front end of the cannula and the front end of the hollow biopsy needle may be brought more close to each other.
The third wheel may be provided with mechanical energy by the third of the mechanical energy transferring means, optionally a mechanical energy transferring means being a flexible shaft providing mechanical energy by means of a rotation of a cable, such as a transmission cable, being part of this mechanical energy transferring means.

Optionally the cannula comprises at least one aperture in its outer surface for removing the tissue sample from the tissue receiving means of the hollow biopsy needle when the tissue receiving means is brought in front of the aperture by axially moving (withdrawing) the hollow biopsy needle inside the cannula. The driving mechanism for axially moving forwards and backwards the hollow biopsy needle is then adapted to bring the receiving element in front of the aperture.

This may especially realised by a medical tool arrangement of which the driving mechanism suitable for enabling and disabling, i.e. preventing, the inner needle from rotating when the first wheel is engaging and rotating the hollow biopsy needle, is suitable for axially moving the hollow biopsy needle and the inner needle simultaneously.

A biopsy needle assembly may further comprise a vacuum unit coupled to the hollow biopsy needle.
The biopsy needle assembly for taking tissue sample is provided with an inner needle having a front end and a back end. This inner needle is located inside and coaxially with the hollow biopsy needle in such a way that the cannula, the hollow biopsy needle and the inner needle are mutually axially moveable with or without rotation. The back end of the inner needle may extend from a cavity of the hollow biopsy needle, at the back end of this hollow biopsy needle. The hollow biopsy needle has a cylindrical back end in which the cavity of the hollow biopsy needle extends. The back end of the inner needle is provided with a cylindrical body, which is moveable inside the cylindrical back end of the hollow biopsy needle. The cylindrical back end and the cylindrical body cooperate to provide a piston-cylinder vacuum unit for creating a lowered air pressure in the cavity when the front end of the hollow biopsy needle is axially moved away from the front end of the inner needle. The stroke of the piston in the piston-cylinder vacuum unit is coaxial with the biopsy needle. This makes for a compact design and reduces the distance between the vacuum unit and the end of the needle to a minimum. This reduces the total air volume between the vacuum unit and the needle tip which in turn means that the vacuum unit only has to exhaust a small volume of air. Further the manufacture of the biopsy needle is made simpler and more easily automated, i.e. reduces labour costs. The system doesn't need other accessories.
As such, the biopsy needle assembly of the medical tool arrangement may comprise a vacuum unit coupled to the hollow biopsy needle, the cannula, hollow biopsy needle and inner needle being mutually axially moveable.

The vacuum unit is thus adapted to create a lower air pressure in the inner cavity when the front end of the hollow biopsy needle is axially moved away from the front end of the inner needle. The vacuum unit is thus located uni-axially with the cannula and the hollow biopsy needle.
As the biopsy needle assembly is inserted up to the tissue to be sampled, the hollow biopsy needle is first axially moved forward into the tissue, optionally by a rotation movement. In this way, the front end of the hollow biopsy needle is axially moved away from the front end of the inner needle. As the inner needle is coupled to the piston of the piston-cylinder vacuum unit, and as the axially forward moving hollow biopsy needle is coupled to the cylinder part of this vacuum unit, the volume within the piston-cylinder vacuum unit increases thus sucking air from the needle and creating an underpressure, suction or vacuum at the moment the biopsy needle is introduced in the tissue. Hence, the suction or vacuum is created automatically and no extra intervention of the physician is necessary. Also the amount of suction or vacuum is adapted to the displacement of the hollow biopsy needle. So the risk of applying too much or too little underpressure is minimised. As the inner needle fills part of the cavity of the hollow biopsy needle, the space in which the suction or vacuum is to be created is further minimized. No tubing from an external vacuum unit nor external recipients are required that may disturb the physician in his or her freedom to move and work.
The cannula may be provided with a cylindrical back end, the outer surface of the cylindrical back of the hollow biopsy needle is provided with screw thread. The inner side of the cylindrical back end of the cannula comprises a screw thread suitable for engaging with the screw thread of the cylindrical back of the hollow biopsy needle. The cannula may be rotatably mounted around the common axis of cannula, hollow biopsy needle and inner needle. The cylindrical back end of the cannula may be provided with a contact wheel for engaging with a counter wheel in order to rotate the cannula. By having the screw threads of outer surface of back end of the hollow biopsy needle and inner side of the cylindrical back end of the cannula, while rotating one of the cannula or the hollow biopsy needle and preventing the other of the cannula or the hollow biopsy needle to rotate simultaneously, the cannula may be axially moved relative to the hollow biopsy needle.
The term "mutually axially moveable" of the cannula, the hollow biopsy needle and the inner needle is to be understood in the sense that the cannula, the hollow biopsy needle and the inner needle may move in axial direction along the common axis, independent from each other when required. Optionally the cannula, the hollow biopsy needle and optionally the inner needle may further rotate round the common axis, independently from each other when required.

Optionally, the inner needle front end is located substantially at the outer end of the hollow biopsy needle when its cylindrical body is at its closest position near the cavity of the hollow biopsy needle. This further reduces the space or volume which is to be brought under vacuum. The inner needle front end, optionally being pointed, is provided out of stainless steel, optionally out of stainless steel of medical grade. The needle point preferentially is made out of stainless steel of medical grade. To locate the point of the needle system, the physician can use clinical palpation, ultrasound, MRI or stereotactic X-rays technology or a combination of these possibilities.

Optionally the cannula comprises at least one aperture in its outer surface for allowing removal the tissue sample from the tissue receiving means of the hollow biopsy needle. The tissue receiving means is brought in front of the aperture by axially withdrawing this hollow biopsy needle within the cannula. In such a way, the tissue once sampled, may be removed from the cannula, without the necessity to remove the cannula out of the tissue or body which is to be sampled, avoiding seeding during repeated insertions. For improving the removal of the tissue sample though the aperture, the hollow biopsy needle is rotatably moveable around the common axis of cannula, hollow biopsy needle and inner needle.
As the removal of the cannula is not necessary, several consecutive samplings may be done with only one insertion of the biopsy needle assembly. In a more preferred embodiment, two apertures are provided having substantially the dimensions of the tissue receiving means. The two apertures may be located in front of each other at two opposed sides of the cannula surface. The apertures are optionally located near the back end of the cannula. When the tissue receiving means is brought in front of this aperture or apertures, and a marker element can be coupled to the tissue receiving means so allow accurate return of the tissue receiving means to the position of the previous sampling. The markers allow exact location of the place where a tissue was sampled.

If the driving mechanism is suitable for axially moving the hollow biopsy needle and the inner needle simultaneously, by axially displacing the hollow biopsy needle and inner needle simultaneously backwards, for bringing the samples tissue at the height of the aperture, the suction or vacuum at the inner needle front end will remain and the sampled tissue will be fixed in its position until it is presented at the aperture.

### Brief description of the drawings

Fig. 1 to Fig. 4 show schematically a biopsy needle assembly and its different elements, which assembly is used to provide a medical tool arrangement for taking tissue samples.
Fig. 5 is a schematically view of a medical tool arrangement for taking tissue sample.
Fig. 6a, Fig. 6b, Fig. 6c and Fig. 6d are schematically views of the consecutive steps to be applied to take a tissue sample using the medical tool arrangement as shown in Fig. 5.
Fig. 7 is a schematically view of a cross-section of a gearbox, being part of a medical tool arrangement for taking tissue samples.
Fig. 8 and Fig. 9 are schematically views of alternative medical tool arrangements for taking tissue sample.
In the different figures, the same reference signs refer to the same or analogous elements.

### Description of illustrative embodiments

The present invention will be described with respect to particular embodiments and with reference to certain drawings but the invention is not limited thereto but only by the claims.

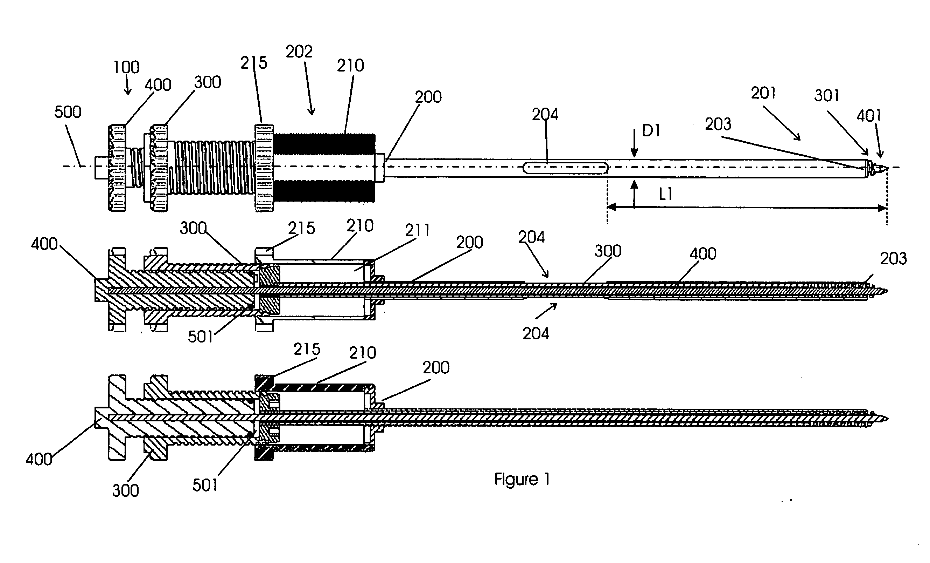

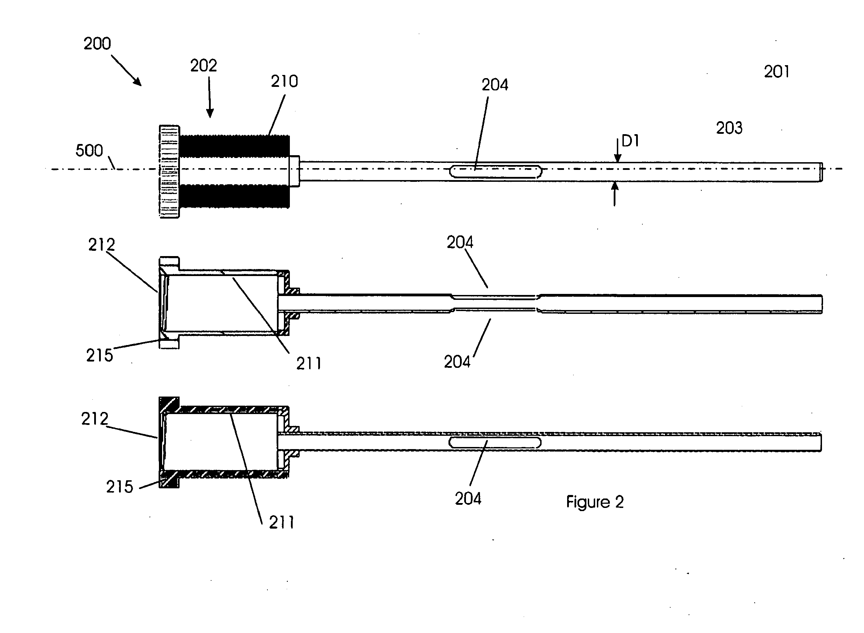

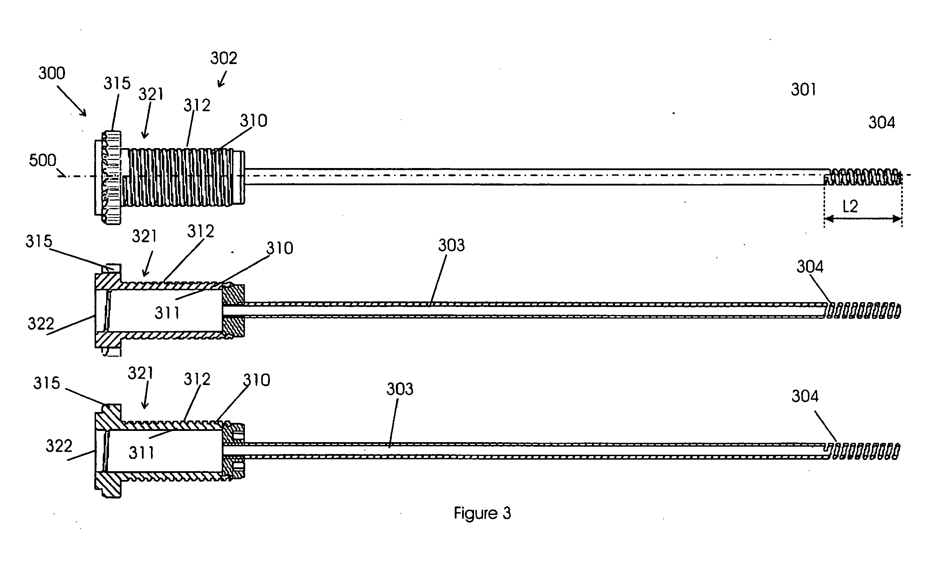

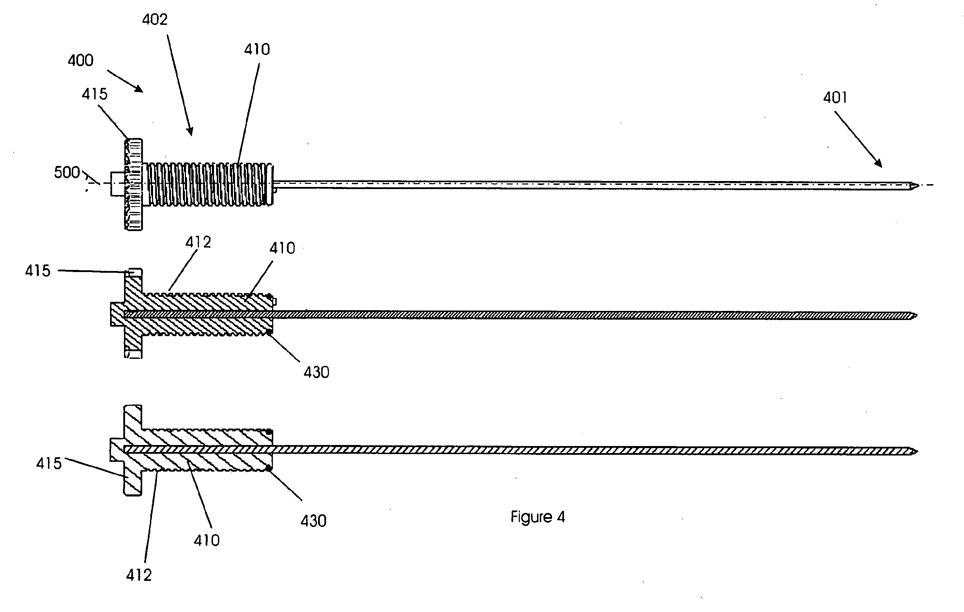

Fig. 1 shows schematically a biopsy needle assembly 100 of a medical tool arrangement. More in particular, a side view and two planar cuts along two mutually perpendicular planes parallel to the axis of the biopsy needle assembly are shown in Figure
1. The biopsy needle assembly comprises three elements: a cannula 200, as shown in similar way in more detail in Fig. 2, a hollow biopsy needle 300 as shown in similar way in more in detail in Fig. 3 and an inner needle 400 as shown in similar way and more in detail in Fig. 4.
   The outer cannula 200 has a front end 201 and a back end 202, the front end being for insertion in a tissue such as a patient tissue. The front end has a cutting edge 203 for cutting a tissue sample. The cannula may comprise at least one, or more than one, apertures 204 for removal of the cut sample by the tissue receiving means 304 of the hollow biopsy needle 300.
   The cannula is provided with a cylindrical back end 210, whose inner side 211 comprises a screw thread 212. The cannula 200 is rotatably mounted around the axis 500 of the biopsy needle assembly. Its cylindrical back end 210 comprises a gear wheel 215 for engaging with a counter gear wheel for making the cannula rotate around the axis 500.
   The hollow biopsy needle 300 is located inside and coaxially with the cannula 200. The hollow biopsy needle 300 has an inner cavity 303 and has a front end 301 and a back end 302. The front end 301 is provided with a tissue receiving means 304. Optionally the tissue receiving means 304 is a spirally shaped tissue receiving means, such as disclosed in US 2003/0114773A1. The hollow biopsy needle 300 has a cylindrical back end 310, and the cavity 303 of the hollow biopsy needle 300 extends into this cylindrical back end. Optionally, that hollow biopsy needle is rotatably mounted around the axis 500 of the biopsy needle assembly 100.
   The outer surface 321 of the cylindrical back end 310 of the hollow biopsy needle 300 is provided with a screw thread 312 for engaging with the screw thread 212 of the inner side 211 of the cylindrical back end 210 of the cannula 200. The cylindrical back end 310 of the hollow biopsy needle 300 is provided with a gear wheel 315 for engaging with a counter gear wheel in order to rotate the hollow biopsy needle around the axis 500. At the inner side 311 of the cylindrical back end 310 of the hollow biopsy needle 300, a screw thread 322 is provided.
   The biopsy needle assembly 100 comprises an inner needle 400 having a front end 401 and a back end 402. The inner needle 400 is located inside and coaxially with the hollow biopsy needle 300. The back end 402 of the inner needle 400 is provided with a cylindrical body 410, which is moveable inside the cylindrical back end 310 of the hollow biopsy needle 300. The inner needle 400 is rotatably mounted around common axis 500 of cannula 200, hollow biopsy needle 300 and inner needle 400
   The cylindrical back end and the cylindrical body make up a piston-cylinder vacuum unit for creating a lowered air pressure in the cavity when the front end of the hollow biopsy needle is axially moved away from the front end of the inner needle and the three elements are mutually axially moveable.
   The cylindrical body 410 has a screw thread 412, for engaging with the screw thread 322 of the the inner side 311 of the cylindrical back end 310 of the hollow biopsy needle 300. The cylindrical body 410 comprises also means 430 to provide an airtight coupling of the cylindrical body 410 and cylindrical back end 310, e.g. a circular sealing member or joint. The cylindrical body 410 is provided with a gear wheel 415 for engaging with a counter gear wheel in order to rotate the inner needle around the axis 500.
   When the inner needle 400 is rotated relative to the hollow biopsy needle 300, the engagement of the screw thread 322 and screw thread 412 forces the inner needle 400 to move backwards from the hollow biopsy needle 300 or the hollow biopsy needle 300 is forced to move forward from the inner needle 400. Thereby, the inner space 501 is increased in volume and the front end 301 of the hollow biopsy needle 300 is axially moved away from the front end 401 of the inner needle 400. As there is an airtight coupling of the cylindrical body 410 and cylindrical back end 310, a vacuum or lowered air pressure is created in the inner space 501. This lowered air pressure is present in the inner cavity 303 as well, as the cavity 303 extends into this inner space 501. This lowered air pressure, present in the inner cavity 303, creates suction or a vacuum at the tissue receiving means 304, more particular at the location where the tissue receiving means 304 and outer end 401 of the inner needle meet. So, the cylindrical back end 310 and the cylindrical body 410 make up a piston-cylinder vacuum unit for creating a lowered pressure in the cavity 303 when the front end 301 of the hollow biopsy needle 300 is moved away axially from the front end 401 of the inner needle 400.

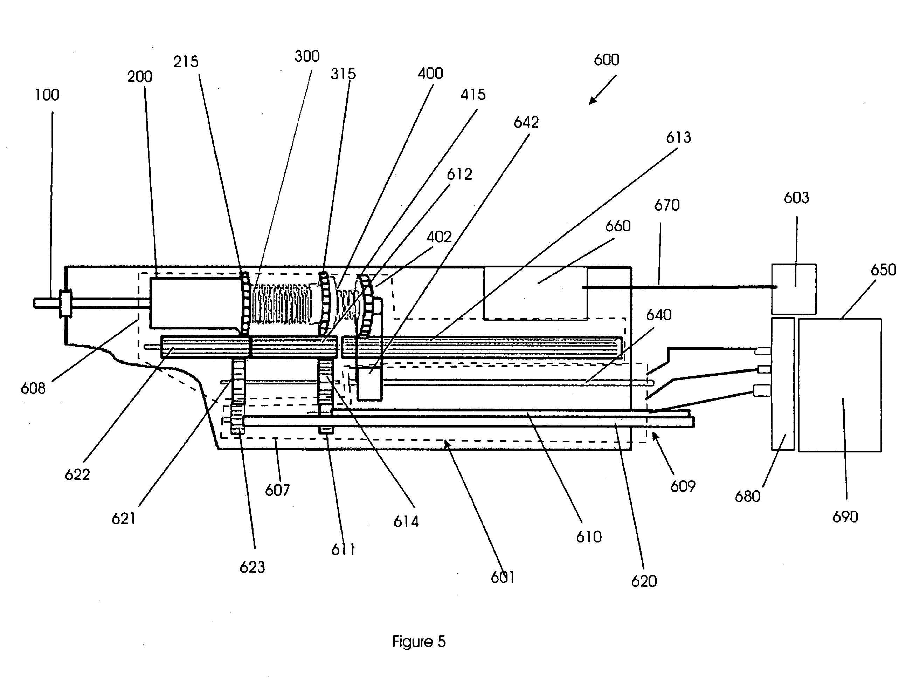

Turning to Figure 5, a medical tool arrangement 600 being a medical tool arrangement for taking a tissue sample by means of a biopsy needle assembly 100 is shown.
The medical tool arrangement 600 comprises a hand-held casing 602. The hand-held casing comprises a tool assembly having a plurality of tools. In this embodiment, the tool assembly is a biopsy needle assembly 100 comprising three biopsy needles, i.e. the cannula 200, the hollow biopsy needle 300 and the inner needle 400, which three biopsy needles are arranged concentrically. The hand-held casing 602 further comprises a driving mechanism 601 for moving the plurality of tools, i.e. the cannula 200, the hollow biopsy needle 300 and the inner needle 400, independently of each other.
The medical tool arrangement is intended for use with a mechanical energy source 650 remote from the hand-held casing 602. The mechanical energy source 650 is couplable to the driving mechanism 610 of the hand-held casing 602 by means of at least a first and a second mechanical energy transferring means, in this particular embodiment three mechanical energy transferring means, in particular three flexible shafts 610, 620 and 640.
One of the mechanical energy transferring means has a length and an axial direction and comprises an energy transferring medium suitable for transferring mechanical energy by making a translational movement longitudinally along its axial direction. The flexible shaft 640 is the mechanical energy transferring means, which means is able to transfer mechanical energy by means of a translation of a transferring medium, such as a transmission cable in the flexible shaft.

The driving mechanism 601 has a means 607 for receiving the mechanical energy transferring means. The means 607 for receiving the mechanical energy transferring means comprises apertures 609 in the hand held casing 602 for allowing the mechanical energy transferring means to be coupled with elements of the drive mechanism at the inside of the hand held casing. The means 607 for receiving the mechanical energy transferring means comprises gear wheels 623 and 611 for receiving and for coupling the mechanical energy transferring means 620 respectively 610. The means 607 for receiving the mechanical energy transferring means comprises brake 642, being e.g. sliding moveable inside the hand held casing, for receiving and for coupling the mechanical energy transferring means 640.
The driving mechanism 601 has means 608 for translating motions of the mechanical energy transferring means 610, 620 and 640 into motions of the plurality of tools. The means 608 for translating motions of the mechanical energy transferring means into motions of the plurality of tools comprises a number of elements such as wheels and/or gear wheels as will be set out hereafter.

The driving mechanism 601 is suitable for axially moving the cannula 200 forwards and backwards in axial direction. To rotate the cannula 200 about the axis, the driving means 601 comprises a gear wheel 622, the latter engaging with the gear wheel 215 of the cylindrical back end 210 of cannula 200. The gear wheel 622 engages a counter gear wheel 621. The counter gear wheel 621 is rotated by the flexible shaft 620, being the first mechanical energy transferring means. This flexible shaft 620 provides the driving mechanism with mechanical energy in the form of rotation of e.g. the transmission cable being part of the flexible shaft. The flexible shaft 620 is coupled to the gear wheel 621 by means of a counter gear wheel 623. Appropriate engaging or blocking of gear wheels, and appropriate cooperating of engaging screw threads, as will be explained below, cause the cannula to move axially forwards and backwards.

The driving mechanism 601 is suitable for moving the hollow biopsy needle 300 axially forwards and backwards. A gear wheel 611 is rotated by rotation of e.g. a transmission cable being part of the flexible shaft 610. The flexible shaft 620 is the second mechanical energy transferring means. Gear wheel 611 is coupled to the gear wheel 315 of the cylindrical back end 310 of the hollow biopsy needle 300 by means of intermediate counter gear wheels 612 and 614. These components, together with appropriate engaging or blocking of gear wheels, and appropriate cooperating of engaging screw threads, as will be explained below, cause the hollow biopsy needle 300 to move forward and backwards in an axial direction as well.
The driving mechanism 601 is suitable for disabling or enabling the inner needle 400 to move in axial direction when hollow biopsy needle 300 is axially moved. For this purpose, a brake or braking means 642 may be provided. When appropriate cooperating of engaging screw threads, as will be explained below, cause the hollow biopsy needle 300 to move forward and backwards in an axial direction, the brake 642 prevents the inner needle from rotating. The brake 642 may also cause the inner needle to be prevented from moving in an axial direction when the hollow biopsy needle is moved axially. For this purpose, the flexible shaft 640 translates the brake 642 backwards or forwards as the case may be, for engaging or disengaging the brake 642 and the back end 402. The flexible shaft 640 is the third mechanical energy transferring means, which means is able to transfer mechanical energy by means of a translation of a transferring medium, such as a transmission cable in the flexible shaft.

The flexible shaft 640 may also move the hollow biopsy needle as well as the inner needle forwards and backwards simultaneously. This may be done by moving the third mechanical energy transferring means further backwards or forwards, when the coupling of the cannula, hollow biopsy needle and inner needle allow such translational movement of the hollow biopsy needle and inner needle simultaneously, as will be set out further.

The driving mechanism is further provided with a gear wheel 613 for engaging the counter gear wheel 415 at the back end of the inner needle 400. This gear wheel may freely rotate about the same axis as the axis of rotation of gear wheel 612. The gear wheel 613 is to guide the inner needle 400 when moved axially forwards or backwards, optionally together with the hollow biopsy needle 300. It also guides the inner needle 400 when the inner needle rotates along with the hollow biopsy needle 300.

The inner needle may be provided as a metal inner needle, e.g. made of stainless steel or a non ferrite metal. It is preferred that the cannula 200, hollow biopsy needle 300 and inner needle 400 as a whole are provided out of stainless steel or other rust-free metal, e.g. medical grade stainless steel.
Optionally, the cylindrical back end 210 and 310 of the cannula and hollow biopsy needle and the cylindrical body 410 of the inner needle are made of a suitable plastic material of which polypropylene is only one example.

The flexible shafts 610, 620 and 640 are coupled to a remote mechanical energy source 650, such as one or more motors. In the embodiment shown in Figure 5, one motor 690 is coupled to the flexible shafts by means of a gearbox 680.
The hand held casing 602 further is provided with a control panel 660 for controlling the drive mechanism 601, more particular for controlling the movements of the mechanical energy transferring means 610, 620 and/or 640. The control signal of the control panel is transmitted to a control unit 603 for activating the first, second and/or third mechanical energy transferring means. In order to transfer the control signals, the medical tool arrangement further comprises a signal transmitting connection 670, which may be a wireless connection or any suitable signal cable. The control unit 603 converts the signal of the control panel 660 to an activation or deactivation of one or more of the mechanical energy transferring means 610, 620 and/or 640.

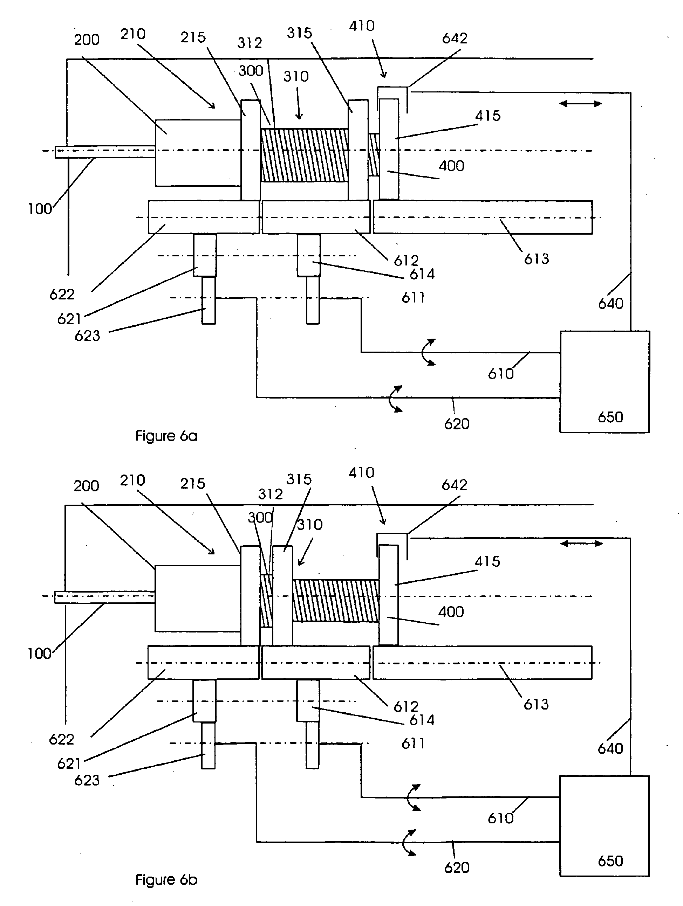

An explanation of how a medical tool arrangement 600 with biopsy needle assembly 100 is applied in order to have a tissue sample cut from an organ or other tissue, is now provided and reference is made to Fig. 6a to Fig. 6d, showing the consecutive steps to be applied to sample a tissue.
In Fig. 6a shows the medical tool arrangement 600 in a starting position. The inner needle 400 is screwed to its position where the cylindrical body 410 is closest to the cavity 303 of hollow biopsy needle 300. The cylindrical back end 310 of the hollow biopsy needle is positioned in such a way that the screw thread 312 and screw thread 212 are engaged, but in a way that only a minor part of the screwing thread 312 is located inside the cylindrical body end 210 of the cannula 200. At the front end of the biopsy needle assembly, the cutting edge 203, the front end 301 of hollow biopsy needle and the front end 401 of the inner needle 400 are substantially flush with each other or coterminous. Together they make a point to penetrate the tissues or organs up to the diseased site where a biopsy has to be taken. Optionally, the front end 401 of inner needle 400 is a sharp point, extending slightly out of the cutting edge 203 and front end 301 of hollow biopsy needle 300.
The biopsy needle assembly is inserted in the tissue to be sampled, by penetrating the front end of the biopsy needle assembly into the tissue, until the tissue to be sampled is level with the front end of the biopsy needle assembly. Scanning equipment such as an MRI scanner, CT scanner, stereotactic radiology equipment or ultrasound scanner may be used to assist in locating the needle correctly.
As shown in Fig. 6b, the hollow biopsy needle only is now brought forward. This is done by rotating the hollow biopsy needle 300 by means of engaging the gear wheel 315 with the rotating counter wheel 612, being driven the mechanical energy transferring means coupled to the gear wheel 612 by means of gear wheels 611 and 614, i.e. the flexible shaft 610. In the meantime, the brake 642 prevents the inner needle from rotating along with the hollow biopsy needle. The brake 642 is closed by appropriate movement of the flexible shaft 640, i.e. by an appropriate translational movement. The engaging screw thread 412 of the inner needle and the screw thread 322 of the cylindrical back end 310 of the hollow biopsy needle 300 force the hollow biopsy needle to move in the direction of the front end. During this movement, the tissue-receiving element 304 is brought, by a screwing action, into the tissue to be sampled.
As such and making reference to features as shown in Figure 1 to 4, the inner space 501 is increased in volume and the front end 301 of the hollow biopsy needle 300 is axially moved away from the front end 401 of the inner needle 400, which is penetrating the tissue to be sampled. Via the cavity 303, a vacuum, created in the inner space 501, is provided at the front end of the biopsy needle assembly, where the tissue to be sampled is sucked by the vacuum into the front end 401 of inner needle 400. The cannula 200 is prevented from rotation along with the hollow biopsy needle 300, because the gear wheel 215 is prevented from rotation by engaging with gear counter wheel 622 which is not driven at that moment but remains in a fixed position. This is done by keeping the flexible shaft 620 in steady position. Because of the engagement of the screw thread 312 at the outer surface of the cylindrical back end 310 with the screw thread 212 at the inner side 211 of the cylindrical back end 210 of the cannula 200, the cylindrical back end 310 of the hollow biopsy needle 300 is actually screwed into the cylindrical back end 210 of the cannula. The final result of this action is shown in Figure 6b.

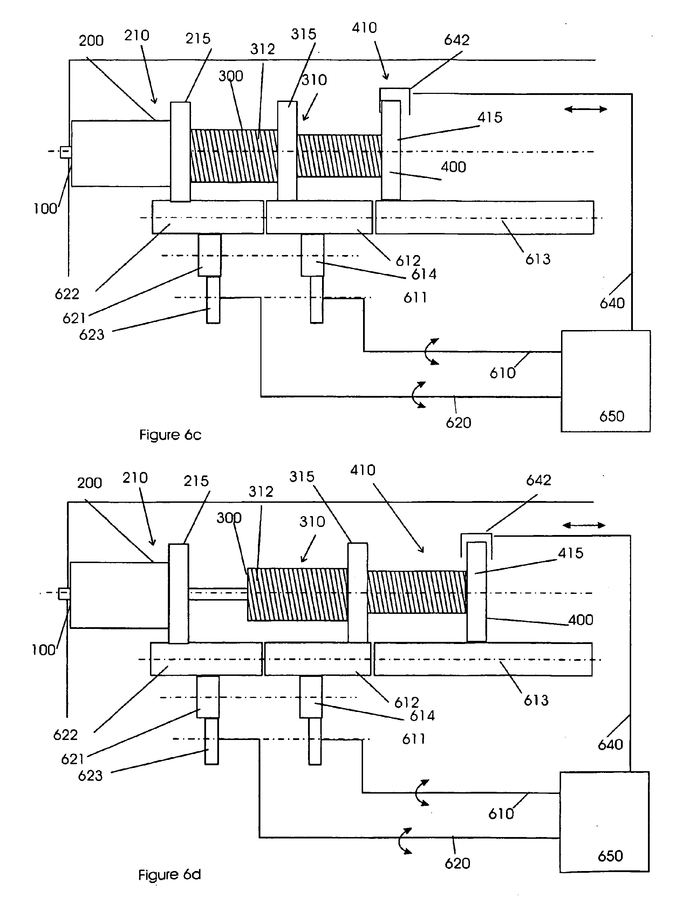

During the next step, as shown in Fig. 6c, the hollow biopsy needle 300 and inner needle 400 are kept in a fixed position by having the gear wheel 315 of the hollow biopsy needle 300 in an engaged position with respect to the counter gear wheel 612, which is not driven. The cannula 200 is rotated by means of the engagement of gear wheel 215 with counter gear wheel 622 driven by the flexible shaft 620. Because of the engagement of the screwing thread 312 at the outer surface of the cylindrical back end 310 with the screw thread 212 at the inner side 211 of the cylindrical back end 210 of the cannula 200, the cannula is axially moved forwards towards the front end of the tissue receiving means 304, meanwhile cutting the tissue sample which is screwed into the screwing shape of the tissue receiving means. As the hollow biopsy needle 300 and inner needle 400 don't change position one with respect to the other, the vacuum which was created, is maintained and the sampled tissue is kept at the front end 401 of inner needle 400.
The cannula is rotated until the screw thread 312 at the outer surface of the cylindrical back end 310 and the screw thread 212 at the inner side 211 of the cylindrical back end 210 of the cannula 200 disengage. The tissue sample is now present in the tissue receiving means 304.
In a next step, as shown in Fig. 6d, the inner needle 400 and hollow biopsy needle 300 are axially moved backwards by the means of appropriate movement of the flexible shaft 640, i.e. by an appropriate translational movement, over such a distance that the sampled tissue in the tissue receiving means is presented at the aperture 204 of the cannula 200. Once the inner needle 400 and hollow biopsy needle 300 are positioned, a minor translation forwards of the flexible shaft 640 cause the brake 642 not to be closed. The inner needle may thus rotate about the common axis of inner needle, hollow biopsy needle and cannula.
When the tissue sample is presented at the aperture 204 of the cannula 200, the hollow biopsy needle 300 and the inner needle 400 may be rotated simultaneously. The gear wheel 315 is driven by means of a rotation provided by the flexible shaft 610, coupled to the gear wheel 315 via gear wheels 611, 614 and 612. The gear wheel 415 at the back end 402 of the inner needle 400 contacts the gear wheel 613 which can freely rotate and which is coaxially mount with gear wheel 612.
During the removal of the tissue sample, the vacuum is interrupted by air, which is allowed to flow into the cavity and into the inner space 501.

In case another consecutive tissue sample is to be taken, the cannula 200, hollow biopsy needle 300 and inner needle 400 are brought again into their starting position and the above steps may be repeated. The cannula, hollow biopsy needle and inner needle are brought back again by a rotating co-axial movement of the inner needle with the hollow biopsy needle with respect to the cannula. Once the cylindrical element of the hollow biopsy needle meets the cylindrical element of the cannula, the cylindrical elements of both engage. The system searches the reference position to repeat the biopsy procedure.

Optionally a marker element, such as a helix, loop or knot with the external diameter smaller than the internal diameter of the cutting cannula and with an expanding or tissue hooking effect in the target tissue, is provided on the tissue receiving means 304 brought at the height of the aperture or apertures 204. By axially moving forward again of the hollow biopsy needle 300 and inner needle 400, the marker can be used to bring the biopsy needle at the position in the tissue, where the sample of the tissue was taken in previous steps.

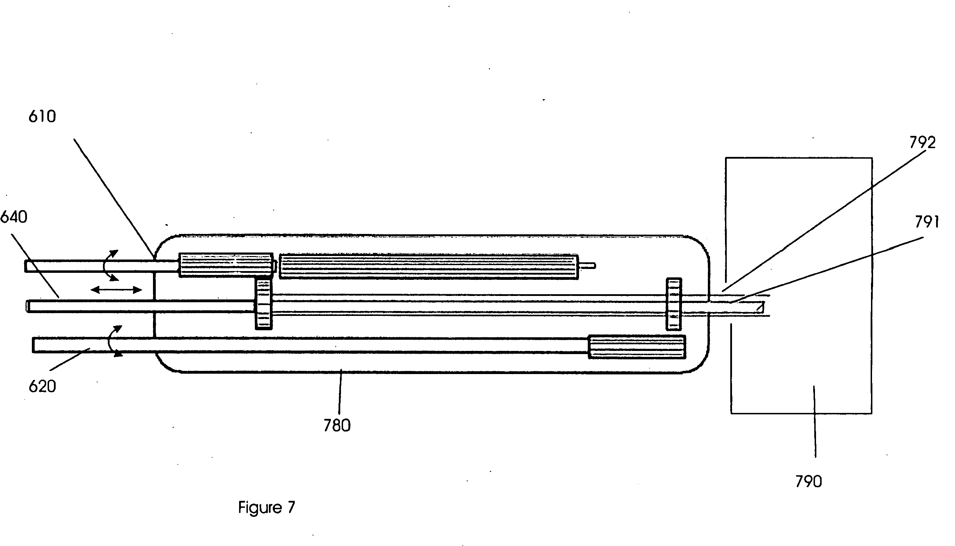

As an example, in the biopsy needle assembly 100 as described by means of the figures 1 to 6, following dimensions are used. The cannula 200 has an inner diameter d1 of about 3 to 4 mm. The length of the biopsy needle assembly which can be brought into the tissue to be sampled, this is the length L1 from the front end of the biopsy needle assembly 100 to the aperture 204, is about 100 mm. The length L2 of the tissue receiving means 304, being optionally substantially equal with the length of the aperture or apertures 204 is about 18 to 20 mm. This length L2 is about the length over which the hollow biopsy needle 300 can move axially as compared to fixed cannula 200 and fixed inner needle 400. The length of the above mentioned cooperating screw threads is about L2. The axial displacement L3 that the cannula 200 is allowed to move is about 26 mm
The length of axial displacement L4 of hollow biopsy needle 300 and inner needle 400, in order to provide the sampled tissue before the aperture 204 is about the maximal insertion length L1 of the biopsy needle assembly. In the embodiment as described, L4 is about 100 mm.
The length of the mechanical energy transferring means, in the embodiment shown in Fig. 1 to 6 being flexible shafts, may be more than 0.5m, such as in the range of 1 m to 2 m.The energy source is coupled to the three mechanical energy transferring means by any suitable means. In a particular case, as shown in Fig. 7, the mechanical energy source 790 has two coaxially mounted elements, i.e. an inner translating element 791 and an outer rotatable element 792.
The coaxially moveable elements are coupled to a gearbox 780 comprising appropriate gear wheel, to transfer their translational or rotational movements into movements of the flexible shafts 610, 620 or 640. The length of the translations made by the inner element 791 may match the length of translation to be made by parts of the biopsy needle assembly 100. The angle of rotation of the inner element 791 may match or may be proportional or even identical to the angle of rotation of the hollow biopsy needle of the biopsy needle assembly 100. The angle of rotation of the outer element 792 may match or may be proportional or even identical to the angle of rotation of the cannula of the biopsy needle assembly 100. Since no additional gear reduction is to be provided, a reduction of the dimensions of the gear wheels to be used in the hand-held casing 600 may be obtained.
As an alternative the mechanical energy source may have three coaxially mounted elements, i.e. an inner translating element, an intermediate element and an outer rotatable element. The angle of rotation of the outer element may match or may be proportional or even identical to the angle of rotation of the cannula of the biopsy needle assembly. The length of the translations made by the inner element may match the length of translation to be made by parts of the biopsy needle assembly. The angle of rotation of the intermediate element may match or may be proportional or even identical to the angle of rotation of the hollow biopsy needle of the biopsy needle assembly.

The at least three mechanical energy transferring means may be present as completely separated means, or may be combined in to one tubular coupling means, in which the at least three mechanical energy transferring means may independently transfer the mechanical energy, by translation and optionally rotation. The at least three mechanical energy transferring means may be combined by providing at least three mechanical energy transferring means parallel to each other in one tubing. Alternatively, at least three mechanical energy transferring means may be provided as at least three coaxial mechanical energy transferring means.
In case the control panel uses a signal transmitting connection 670, being a signal cable, this cable may be integrated in the tubular coupling means as well.

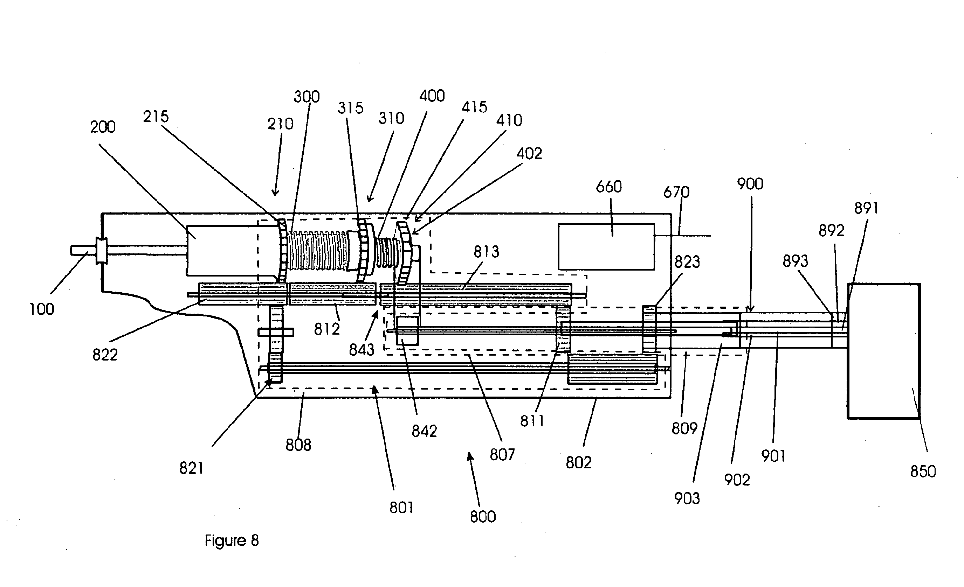

Another embodiment of a medical tool assembly 800 for taking tissue sample is shown in Fig. 8. A mechanical energy source 850 has three coaxially mounted elements, i.e. an inner translating element 891, an intermediate and rotatable element 892 and an outer rotatable element 893. The medical tool assembly 800 is coupled to the three coaxially mounted elements by means of a multilayered coaxial flexible shaft 900.
A driving mechanism 801 of the medical tool assembly 800 has a means 807 for receiving the mechanical energy transferring means. The means 807 for receiving the mechanical energy transferring means comprises connector 809 on the hand held casing 602 for allowing the mechanical energy transferring means to be coupled with elements of the drive mechanism at the inside of the hand held casing. The means 807 for receiving the mechanical energy transferring means comprises gear wheels 823 and 811 for receiving and for coupling the mechanical energy transferring means 903 respectively 902. The means 807 for receiving the mechanical energy transferring means comprises brake 842, being e.g. sliding moveable inside the hand held casing, for receiving and for coupling the mechanical energy transferring means 901.
The driving mechanism 801 has means 808 for translating motions of the mechanical energy transferring means 901, 902 and 903 into motions of the plurality of tools. The means 808 for translating motions of the mechanical energy transferring means into motions of the plurality of tools comprises a number of elements such as wheels and/or gear wheels as will be set out hereafter.

The inner part or layer 901 of the multilayered flexible shaft 900 is coupled to the translating inner element 891 for transferring the translational movement. The intermediate part or layer 902 of the flexible shaft 900 is coupled to the rotating and translating intermediate and rotational element 892 for transferring the movements of the intermediate element 892. The outer part or layer 903 of the flexible shaft 900 is coupled to the rotating and translating outer and rotational element 893 for transferring the movements of the intermediate element 893.
The medical tool assembly 800 comprises a hand held casing 802 comprising the biopsy needle assembly 100 and a driving mechanism 801. The driving mechanism 801 is suitable for axially moving the cannula 200 forwards and backwards in axial direction. To rotate the cannula 200 about the axis, the driving means 801 comprises gear wheel 822, the latter engaging with the gear wheel 215 of the cylindrical back end 210 of cannula 200. The gear wheel 822 is coupled to a gear wheel 823 by means of a suitable set 821 of gear wheels, transferring the rotation of the outer part or layer 903 of the flexible shaft 900 to the gear wheel 822, hence to cannula 200. The flexible shaft 903 is coupled to the gear wheel 821 by means of a counter gear wheel 823
The outer part or layer 903 of the flexible shaft 900 is the first mechanical energy transferring means. The outer part or layer 903 of the flexible shaft 900 provides the driving means with mechanical energy in the form of rotation of the flexible shaft.

Appropriate engaging or blocking of gear wheels, and appropriate cooperating of engaging screw threads, being applied in a similar way as set out above in relation to the medical tool arrangement 600 of Fig. 5, causes the cannula to move axially forwards and backwards.

The driving mechanism 801 is suitable for moving the hollow biopsy needle 300 axially forwards and backwards. A gear wheel 811 is rotated by the intermediate part or layer 902 of the flexible shaft 900. The intermediate part or layer 902 is the second mechanical energy transferring means. Gear wheel 811 is coupled to the gear wheel 315 of the cylindrical back end 310 of the hollow biopsy needle 300 by means of intermediate counter gear wheels 812 and 813. These components, together with appropriate engaging or blocking of gear wheels, and appropriate cooperating of engaging screw threads, as will be explained below, causes the hollow biopsy needle 300 to move forward and backwards in an axial direction as well.
The driving mechanism 801 is suitable for disable or enable inner needle 400 to move in axial direction when hollow biopsy needle 300 is axially moved. For this purpose a brake or braking means 842 may be provided. Appropriate cooperation of engaging screw threads causes the hollow biopsy needle 300 to move forward and backwards in an axial direction while the brake 842 prevents the inner needle from rotating. The brake 842 also causes the inner needle to be prevented from moving in an axial direction when the hollow biopsy needle is moved axially. The counter gear wheel is positioned at the gap 843 present between the gear wheels 812 and 813. The gap 843 is wide enough to prevent the wheel 415 to contact wheels 812 and 813. The brake keeps the inner needle locked, i.e. prevents the inner needle from axially moving. For this purpose, the inner part or layer 901 of the multilayered flexible shaft 900 translates the brake 842 backwards or forwards as the case may be, for engaging or disengaging the brake 842 with the back end 402. The inner part or layer 901 of the multilayered flexible shaft 900 is the third mechanical energy transferring means, which means is able to transfer mechanical energy by means of a translation of a transferring medium, such as a transmission cable in the flexible shaft. The gear wheel 315 of the hollow biopsy needle 300 is engaged and driven by the intermediate part or layer 902, while the outer part or layer 903 is kept steady. Due to the engagement of the screw thread 312 at the outer surface of the cylindrical back end 310 with the screw thread 212 at the inner side 211 of the cylindrical back end 210 of the cannula 200, the cylindrical back end 310 of the hollow biopsy needle 300 is actually screwed into the cylindrical back end 210 of the cannula.
The brake 842 prevents the inner needle 400 to rotate along with the hollow biopsy needle, and the wheel 415 is located at the gap 843 between the wheels 812 and 813. The engaging screw thread 412 of the inner needle and the screw thread 322 of the cylindrical back end 310 of the hollow biopsy needle 300 allow the hollow biopsy needle to move in the direction of the front end relative to the inner needle 400, while the inner needle is prevented to move along with the hollow biopsy needle.

The inner part or layer 901 of the multilayered flexible shaft 900 may also move the hollow biopsy needle as well as the inner needle forwards and backwards simultaneously. This may be done by moving the third mechanical energy transferring means further backwards or forwards, when the coupling of the cannula, hollow biopsy needle and inner needle allow such translational movement of the hollow biopsy needle and inner needle simultaneously.

The gear wheel 813 engages the counter gear wheel 415 at the back end of the inner needle 400. This gear wheel may rotate about the same axis as the axis of rotation of gear wheel 812. The gear wheel 813 is to guide the inner needle 400 when moved axially forwards or backwards optionally together with the hollow biopsy needle 300. It also guides the inner needle 400 when the inner needle rotates along with the hollow biopsy needle 300.
The working of the medical tool arrangement as shown in Fig. 8 is similar to the working of the medical tool arrangement as shown in Fig. 5. The consecutive steps to take a tissue sample using the medical tool arrangement 1600 are similar as shown in Fig. 6a to Fig. 6d.

The hand held casing 602 further is provided with a control panel 660 for controlling the drive mechanism 601, similar as set out for the medical tool arrangement 600 of Fig. 5.

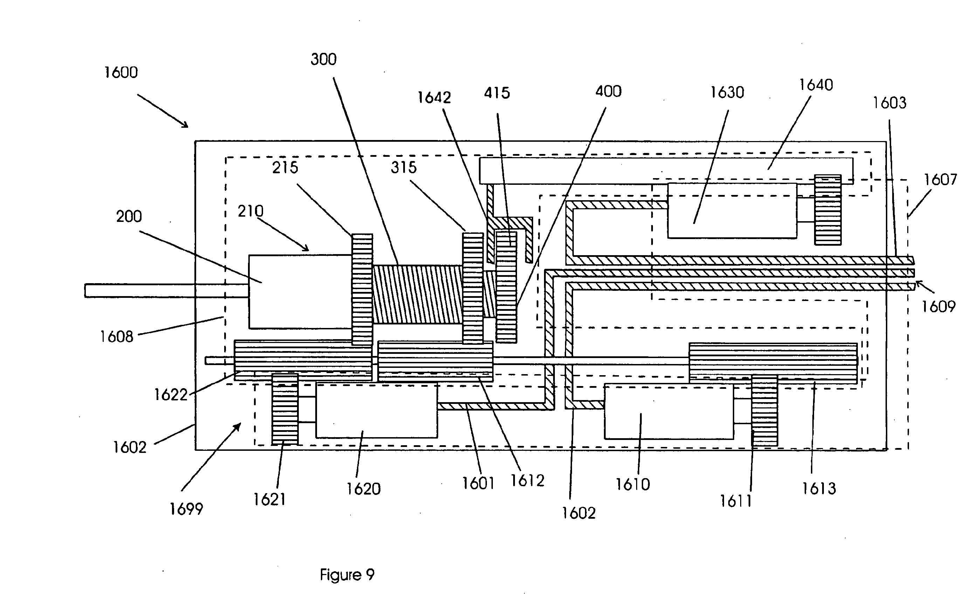

It is understood that as an alternative to the above mentioned flexible shafts 610, 620 and 640, some or all of the flexible shafts may be replaced by compressed gas tubes, being mechanical energy transferring means. The energy transferring medium being compressed gas may move back and forward in the compressed gas tubes in order to transfer mechanical energy from an energy source to the hand-held casing. The hand-held casing is additionally provided with a means to translate expansion of compressed gas from one of the compressed gas tubes into a rotation of a corresponding gear wheel or a translation of the brake.
Similarly, as another alternative to the above mentioned flexible shafts 610, 620 and 640, some or all of the flexible shafts may be replaced by hydraulic tubes, being mechanical energy transferring means. The energy transferring medium being hydraulic fluid may move back and forward in the hydraulic tubes in order to transfer mechanical energy from an energy source to the hand-held casing. The hand-held casing is additionally provided with a means to translate hydraulic energy of the hydraulic fluid from one of the hydraulic tubes into a rotation of a corresponding gear wheel or a translation of the brake.
In both cases of compressed gas tubes or hydraulic tubes, the energy source provides energy transferring medium to the hand-held casing via appropriate tubing, optionally through an appropriate valve system. The opening or closing of the valves in the valve system is controlled by the control unit upon signals of the control panel.
A medical tool arrangement 1600 is provided with three mechanical energy transferring means suitable for transferring mechanical energy by making a translational movement, and using compressed gas as energy transferring medium is shown in Fig. 9.

The medical tool arrangement 1600 comprises a biopsy needle assembly 100 and a hand held casing 1602 similar as the medical tool arrangements 600 and 800.
The medical tool arrangement 1600 comprises a driving mechanism 1699 for axially moving the cannula 200 forwards and backwards.
A driving mechanism 1699 of the medical tool assembly 1600 has a means 1607 for receiving the mechanical energy transferring means. The means 1607 for receiving the mechanical energy transferring means comprises one or more apertures 1609 in the hand held casing 1602 for allowing the mechanical energy transferring means to be coupled with elements of the drive mechanism at the inside of the hand held casing. The means 1607 for receiving the mechanical energy transferring means comprises a first driving means, e.g. a motor 1620, which drives gear wheel 1621, a second driving means, e.g. a motor 1610, which drives gear wheel 1611 and a third driving means, which may be a motor 1630. Each of the driving means is coupled to one of the mechanical energy transferring means 1601, 1602 and 1603.

The driving mechanism 1699 has means 1608 for translating motions of the mechanical energy transferring means 1601, 1602 and 1603into motions of the plurality of tools. The means 1608 for translating motions of the mechanical energy transferring means into motions of the plurality of tools comprises a number of elements such as wheels and/or gear wheels as will be set out hereafter.
This driving mechanism 1699 comprises a driving means, e.g. a motor 1620, which drives gear wheel 1621 and counter gear wheel 1622, the latter engaging with the gear wheel 215 of the cylindrical back end 210 of cannula 200. The motor 1620 is powered by the first mechanical energy transferring means 1601 suitable for transferring mechanical energy by making a translational movement using compressed gas as energy transferring medium.
Appropriate engaging or blocking of gear wheels, and appropriate cooperating of engaging screw threads, similar as already set out above with regard to Fig. 6a to 6d, causes the cannula to move axially forwards and backwards.

The driving mechanism 1699 is suitable for moving the hollow biopsy needle 300 axially forwards and backwards. The driving mechanism 1699 comprises a driving means, e.g. a motor 1610, which drives a gear wheel 1611, coupled to only the gear wheel 315 of the cylindrical back end 310 of the hollow biopsy needle 300 by means of a first counter gear wheel 1612, or which may engage with the gear wheel 315 of the cylindrical back end 310 of the hollow biopsy needle 300 and the gear wheel 415 of the inner needle 400 by means of a second counter gear wheel 1613. The motor 1610 is powered by the second mechanical energy transferring means 1602 suitable for transferring mechanical energy by making a translational movement using compressed gas as energy transferring medium.
The driving mechanism 1699 is suitable for preventing inner needle 400 to move in axial direction when hollow biopsy needle is axially moved. For this purpose a brake or braking means 1642 may be provided. Appropriate cooperation of engaging screw threads causes the hollow biopsy needle 300 to move forward and backwards in an axial direction. A brake 1642 prevents the inner needle 400 from rotating may also cause the inner needle 400 to be prevented from moving in an axial direction when the hollow biopsy needle 300 is moved axially.
The brake or braking means 1642 may be powered by a driving means, which may be a motor 1630, which by means of a set of gear wheels and a geared belt 1640, may move the hollow biopsy needle as well as the inner needle forward and backward. These components, together with appropriate engaging or blocking of gear wheels, and appropriate cooperating of engaging screw threads, causes the hollow biopsy needle 300 to move forward and backwards in an axial direction as well. The motor 1630 is powered by the third mechanical energy transferring means 1603 suitable for transferring mechanical energy by making a translational movement using compressed gas as energy transferring medium.

The working of the medical tool arrangement 1600 as shown in Fig. 9 is similar to the working of the medical tool arrangement as shown in Fig. 5. The step of axially moving backwards the inner needle 400 and hollow biopsy needle 300 is done by the means of a set of gear wheels and a timing or geared belt 1640. The timing or gear belt 1640 is driven over such a distance that the sampled tissue in the tissue receiving means is presented at the aperture 204 of the cannula 200, meanwhile bringing gear wheels 315 and 415 in engagement with counter gear wheel 1613. By rotation of the gear wheel 1613, the inner needle 400 and hollow biopsy needle 300 is rotated simultaneously to line up with the aperture and the tissue can be removed from the receiving means 304. During the removal of the tissue sample, the vacuum is interrupted by air, which is allowed to flow into the cavity and into the inner space 501.

## Claims

1. Medical tool arrangement (600) adapted to take tissue sample comprising a hand-held casing (602), the hand-held casing comprising a driving mechanism (601), the medical tool arrangement is adapted to be used with a mechanical energy source (650) remote from the hand-held casing (602), the mechanical energy source (650) being adapted to be coupled to the driving mechanism (610) of the hand-held casing (602) by means of at least a first and a second mechanical energy transferring means (610,620,640), wherein at least one of the first, and second mechanical energy transferring means has a length and an axial direction and comprises an energy transferring medium adapted to transfer mechanical energy by making a translational movement longitudinally along its axial direction, **characterised by** the hand-held casing comprising a biopsy needle assembly (100) having a plurality of biopsy needles, said drive mechanism is adapted to move said plurality of biopsy needles independently of each other, wherein, the biopsy needles comprise:
- an outer cannula (200) having a front end (201) and a back end (202), the front end being for insertion in a tissue, the front end having a cutting edge (203) adapted to cut a tissue sample;
- a hollow biopsy needle (300) located inside and coaxially with the cannula (200), the hollow biopsy needle (300) having an inner cavity (303) and having a front end (301) and a back end (302), the front end (301) being provided with a tissue receiving means (304), the tissue receiving means (304) cooperating with the cutting edge (203) of cannula (200) for receiving a cut tissue sample;
- an inner needle (400) having a front end (401) and a back end (402), the inner needle (400) being located inside and coaxially with the hollow biopsy needle (300).

2. Medical tool arrangement according to claim 1, wherein the driving mechanism (601) is adapted for:
o at least axially displacing forwards and backwards the cannula (200) relative to the hollow biopsy needle (300) and the inner needle (400);
o at least axially displacing forwards and backwards the hollow biopsy needle (300) relative to the cannula (200) and the inner needle (400)
o enabling or disabling the inner needle (400) to move in axial direction along with the hollow biopsy needle (300) when the hollow biopsy needle (300) is axially moved.

3. Medical tool arrangement according to any one of the claims 1 to 2, wherein the driving mechanism (601) comprises a driving means for enabling or disabling the inner needle (400) to move in axial direction along with the hollow biopsy needle (300) when the hollow biopsy needle (300) is axially moved, the at least one of the first and second mechanical energy transferring means being suitable for transferring mechanical energy by making a translational movement is for providing mechanical energy to the driving means (601) for enabling or disabling the inner needle (400) to move in axial direction along with the hollow biopsy needle (300) when the hollow biopsy needle (300) is axially moved.

4. Medical tool arrangement according to any previous claim, wherein the at least one of the first, and second and optionally a third mechanical energy transferring means suitable for transferring mechanical energy by making a translational movement is a flexible shaft.

5. Medical tool arrangement according to claim 4, wherein the first, second and third mechanical energy transferring means are three flexible shafts (610, 620, 640).

6. Medical tool arrangement according to any of the previous claims, wherein the at least one of the first and second and optionally a third mechanical energy transferring means being suitable for transferring mechanical energy by making a translational movement is a hydraulic tube comprising a hydraulic fluid.

7. Medical tool arrangement according to claim 6, wherein the first, second and third mechanical energy transferring means are three hydraulic tubes comprising a hydraulic fluid.

8. Medical tool arrangement according to any previous claim, wherein the at least one of the first and second and optionally a third mechanical energy transferring means being suitable for transferring mechanical energy by making a translational movement is a compressed gas tube suitable for conducting compressed gas.

9. Medical tool arrangement according to claim 8, wherein the first, second and third mechanical energy transferring means are three compressed gas tubes suitable for conducting compressed gas.

10. Medical tool arrangement according to any one of the claims 1 to 9, wherein the hand-held casing (602) further comprises a control panel (660) for controlling the driving mechanism (601).

11. Medical tool arrangement according to claim 10, wherein the mechanical energy source comprises a control unit (603) for activating the first, second and optionally a third mechanical energy transferring means, the medical tool arrangement further comprising a signal transmitting connection (670) between the control panel (660) and the control unit (603) for converting the signal of the control panel (660) to activation or deactivation of one or more of the mechanical energy transferring means (610, 620, 640).

## Patentansprüche

1. Medizinische Werkzeuganordnung (600), die zur Entnahme einer Gewebeprobe ausgebildet ist, umfassend ein tragbares Gehäuse (602), wobei das tragbare Gehäuse einen Antriebsmechanismus (601) umfasst, wobei die medizinische Werkzeuganordnung zur Verwendung mit einer mechanischen Energiequelle (650) fern dem tragbaren Gehäuse (602) ausgebildet ist, wobei die mechanische Energiequelle (650) zur Kopplung an den Antriebsmechanismus (610) des tragbaren Gehäuses (602) mit Hilfe mindestens eines ersten und eines zweiten mechanische Energie übertragenden Mittels (610, 620, 640) ausgebildet ist, wobei mindestens eines von dem ersten und zweiten mechanische Energie übertragenden Mittel eine Länge und eine Axialrichtung aufweist und ein Energieübertragungsmediam umfasst, das zur Übertragung von mechanischer Energie ausgebildet ist, indem es eine Translationsbewegung in Längsrichtung entlang seiner Axialrichtung ausführt, **dadurch gekennzeichnet, dass** das tragbare Gehäuse eine Biopsienadelanordnung (100) mit einer Mehrzahl von Biopsienadeln umfasst, wobei der Antriebsmechanismus zum Bewegen der Mehrzahl von Biopsienadeln unabhängig voneinander ausgebildet ist, wobei die Biopsienadeln umfassen:
- eine äußere Kanüle (200) mit einem vorderen Ende (201) und einem hinteren Ende (202), wobei das vordere Ende zum Einsetzen in ein Gewebe dient, das vordere Ende eine Schneidkante (203) aufweist, die zum Schneiden einer Gewebeprobe ausgebildet ist;
- eine hohle Biopsienadel (300), die sich im Inneren und koaxial mit der Kanüle (200) befindet, wobei die hohle Biopsienadel (300) einen inneren Hohlraum (303) aufweist und ein vorderes Ende (301) und ein hinteres Ende (302) aufweist, wobei das vordere Ende (301) mit einem Gewebeaufnahmemittel (304) bereitgestellt ist, wobei das Gewebeaufnahmemittel (304) mit der Schneidkante (203) der Kanüle (200) zur Aufnahme einer geschnittenen Gewebeprobe zusammenwirkt;
- eine innere Nadel (400) mit einem vorderen Ende (401) und einem hinteren Ende (402), wobei die innere Nadel (400) im Inneren und koaxial mit der hohlen Biopsienadel (300) liegt.

2. Medizinische Werkzeuganordnung nach Anspruch 1, wobei der Antriebsmechanismus (601) ausgebildet ist zum:
o zumindest axialen Vorwärts- und Rückwärtsverschieben der Kanüle (200) relativ zu der hohlen Biopsienadel (300) und der inneren Nadel (400);
o zumindest axialen Vorwärts- und Rückwärtsverschieben der hohlen Biopsienadel (300) relativ zu der Kanüle (200) und der inneren Nadel (400);
o Freigeben oder Sperren der Bewegung der inneren Nadel (400) in axialer Richtung gemeinsam mit der hohlen Biopsienadel (300), wenn die hohle Biopsienadel (300) axial bewegt wird.

3. Medizinische Werkzeuganordnung nach einem der Ansprüche 1 bis 2, wobei der Antriebsmechanismus (601) ein Antriebsmittel zum Freigeben oder Sperren der Bewegung der inneren Nadel (400) in axialer Richtung gemeinsam mit der hohlen Biopsienadel (300), wenn die hohle Biopsienadel (300) axial bewegt wird, umfasst, wobei das mindestens eine des ersten und zweiten mechanische Energie übertragenden Mittels, das zur Übertragung mechanischer Energie geeignet ist, indem es eine Translationsbewegung ausführt, zur Bereitstellung mechanischer Energie für das Antriebsmittel (601) dient, zum Freigeben oder Sperren der Bewegung der inneren Nadel (400) in axialer Richtung gemeinsam mit der hohlen Biopsienadel (300), wenn die hohle Biopsienadel (300) axial bewegt wird.

4. Medizinische Werkzeuganordnung nach einem der vorangehenden Ansprüche, wobei das mindestens eine von dem ersten und zweiten und gegebenenfalls dritten mechanische Energie übertragenden Mittel, das zur Übertragung mechanischer Energie geeignet ist, indem es eine Translationsbewegung ausführt, ein biegsamer Schaft ist.

5. Medizinische Werkzeuganordnung nach Anspruch 4, wobei das erste, zweite und dritte mechanische Energie übertragende Mittel drei biegsame Schäfte (610, 620, 640) sind.

6. Medizinische Werkzeuganordnung nach einem der vorangehenden Ansprüche, wobei das mindestens eine von dem ersten und zweiten und gegebenenfalls dritten mechanische Energie übertragenden Mittel, das zur Übertragung mechanischer Energie geeignet ist, indem es eine Translationsbewegung ausführt, ein hydraulischer Schlauch ist, der ein hydraulisches Fluid umfasst.

7. Medizinische Werkzeuganordnung nach Anspruch 6, wobei das erste, zweite und dritte mechanische Energie übertragende Mittel drei hydraulische Schläuche sind, die ein hydraulisches Fluid umfassen.

8. Medizinische Werkzeuganordnung nach einem der vorangehenden Ansprüche, wobei das mindestens eine von dem ersten und zweiten und gegebenenfalls dritten mechanische Energie übertragenden Mittel, das zur Übertragung mechanischer Energie geeignet ist, indem es eine Translationsbewegung ausführt, ein Druckgasschlauch ist, der zum Leiten von Druckgas geeignet ist.

9. Medizinische Werkzeuganordnung nach Anspruch 8, wobei das erste, zweite und dritte mechanische Energie übertragende Mittel drei Druckgasschläuche sind, die zum Leiten von Druckgas geeignet sind.

10. Medizinische Werkzeuganordnung nach einem der Ansprüche 1 bis 9, wobei das tragbare Gehäuse (602) des Weiteren eine Steuertafel (660) zum Steuern des Antriebsmechanismus (601) umfasst.

11. Medizinische Werkzeuganordnung nach Anspruch 10, wobei die mechanische Energiequelle eine Steuereinheit (603) zum Aktivieren des ersten und zweiten und gegebenenfalls dritten mechanische Energie übertragenden Mittels umfasst, wobei die medizinische Werkzeuganordnung des Weiteren eine Signalübertragungsverbindung (670) zwischen der Steuertafel (660) und der Steuereinheit (603) zum Umwandeln des Signals der Steuertafel (660) für die Aktivierung oder Deaktivierung eines oder mehrerer mechanische Energie übertragenden Mittels beziehungsweise übertragender Mittel (610, 620, 640) umfasst.

## Revendications

1. Agencement d'outil médical (600) conçu pour prendre un échantillon de tissu comprenant un boîtier portatif (602), le boîtier portatif comprenant un mécanisme d'entraînement (601), l'agencement d'outil médical est conçu pour être utilisé avec une source d'énergie mécanique (650) distante du boîtier portatif (602), la source d'énergie mécanique (650) étant conçue pour être couplée au mécanisme d'entraînement (610) du boîtier portatif (602) au moyen d'au moins un premier et d'un deuxième moyen de transfert d'énergie mécanique (610, 620, 640), dans lequel au moins un des premier et deuxième moyens de transfert d'énergie mécanique a une certaine longueur et une direction axiale et comprend un support de transfert d'énergie conçu pour transférer une énergie mécanique en effectuant un mouvement de translation de façon longitudinale le long de sa direction axiale, **caractérisé par** le boîtier portatif comprenant un ensemble d'aiguilles de biopsie (100) ayant une pluralité d'aiguilles de biopsie, ledit mécanisme d'entraînement est conçu pour déplacer ladite pluralité d'aiguilles de biopsie indépendamment les unes des autres, dans laquelle, les aiguilles de biopsie comprennent :
- une canule extérieure (20C) ayant une extrémité avant (201) et une extrémité arrière (202), l'extrémité avant servant à l'insertion dans un tissu, l'extrémité avant ayant un bord de coupe (20) conçu pour couper un échantillon de tissu ;
- une aiguille de biopsie creuse (300) située à l'intérieur de la canule (200) et coaxiale avec cette dernière, l'aiguille de biopsie creuse (300) ayant une cavité intérieure (303) et ayant une extrémité avant (301) et une extrémité arrière (302), l'extrémité avant (301) étant munie d'un moyen de réception de tissu (304), le moyen de réception de tissu (304) coopérant avec le bord de coupe (203) de la canule (200) pour recevoir un échantillon de tissu couplé ;
- une aiguille intérieure (400) ayant une extrémité avant (401) et une extrémité arrière (402), l'aiguille intérieure (400) étant située à l'intérieur de l'aiguille de biopsie creuse (300) et coaxiale avec cette dernière.

2. Agencement d'outil médical selon la revendication 1, dans lequel le mécanisme d'entraînement (601) est conçu pour :
- au moins déplacer de façon axiale en avant et en arrière la canule (200) par rapport à l'aiguille de biopsie creuse (300) et à l'aiguille intérieure (400) ;
- au moins déplacer de façon axiale en avant et en arrière l'aiguille de biopsie creuse (300) par rapport à la canule (200) et à l'aiguille intérieure (40C) ;
- permettre ou interdire à l'aiguille intérieure (400) de se déplacer dans la direction axiale en même temps que l'aiguille de biopsie creuse (300) quand l'aiguille de biopsie creuse (300) est déplacée de façon axiale.

3. Agencement d'outil médical selon l'une quelconque des revendications 1 à 2, dans lequel le mécanisme d'entraînement (601) comprend un moyen d'entraînement pour permettre ou interdire à l'aiguille intérieure (400) de se déplacer dans la direction axiale en même temps que l'aiguille de biopsie creuse (300) quand l'aiguille de biopsie creuse (300) est déplacée de façon axiale, l'au moins un des premier et deuxième moyens de transfert d'énergie mécanique étant approprié pour transférer une énergie mécanique en effectuant un mouvement de translation pour fournir une énergie mécanique au moyen d'entraînement (601) pour permettre ou interdire à l'aiguille intérieure (400) de se déplacer dans la direction axiale en même temps que l'aiguille de biopsie creuse (300) quand l'aiguille de biopsie creuse (300) est déplacée de façon axiale.

4. Agencement d'outil médical selon l'une quelconque des revendications précédentes, dans lequel l'au moins un des premier et deuxième et de manière facultative troisième moyens de transfert d'énergie mécanique appropriés pour transférer une énergie mécanique en effectuant un mouvement de translation est un arbre souple.

5. Agencement d'outil médical selon la revendication 4, dans lequel les premier, deuxième et troisième moyens de transfert d'énergie mécanique sont trois arbres souples (610, 620, 640).

6. Agencement d'outil médical selon l'une quelconque des revendications précédentes, dans lequel l'au moins un des premier et deuxième et de manière facultative troisième moyens de transfert d'énergie mécanique approprié pour transférer une énergie mécanique en effectuant un mouvement de translation est un tube hydraulique comprenant un fluide hydraulique.

7. Agencement d'outil médical selon la revendication 6, dans lequel les premier, deuxième et troisième moyens de transfert d'énergie mécanique sont trois tubes hydrauliques comprenant un fluide hydraulique.

8. Agencement d'outil médical selon l'une quelconque des revendications précédentes, dans lequel l'au moins un des premier et deuxième et de manière facultative troisième moyens de transfert d'énergie mécanique appropriés pour transférer une énergie mécanique en effectuant un mouvement de translation est un tube à gaz comprimé approprié pour conduire un gaz comprimé.

9. Agencement d'outil médical selon la revendication 8, dans lequel les premier, deuxième et troisième moyens de transfert d'énergie mécanique sont trois tubes à gaz comprimé appropriés pour conduire un gaz comprimé.

10. Agencement d'outil médical selon l'une quelconque des revendications 1 à 9, dans lequel le boîtier portatif (602) comprend en outre un panneau de commande (660) pour commander le mécanisme d'entraînement (601).

11. Agencement d'outil médical selon la revendication 10, dans lequel la source d'énergie mécanique comprend une unité de commande (603) pour activer les premier, deuxième et de manière facultative troisième moyens de transfert d'énergie mécanique, l'agencement d'outil médical comprenant en outre une connexion d'émission de signal (670) entre le panneau de commande (660) et l'unité de commande (603) pour transformer le signal du panneau de commande (660) en activation ou désactivation d'un ou plusieurs des moyens de transfert d'énergie mécanique (610, 620, 640).
